# EUROPEAN PATENT SPECIFICATION

(11) **EP 2 898 903 B1**
(45) Date of publication and mention of the grant of the patent: **28.12.2016**
(21) Application number: 13850943.5
(22) Date of filing: 02.09.2013
(51) Int. Cl.: A61L 15/60, A61L 15/42, A61L 15/28, A61L 15/26, A61L 15/32, A61L 15/44, A61F 13/00, A61L 15/22, B65B 55/08, B65B 55/16, B65B 5/04

(54) **HIGH HYGROSCOPIC WOUND DRESSING AND PREPARATION METHOD AND USE THEREOF**
HOCHHYGROSKOPISCHER WUNDVERBAND UND HERSTELLUNGSVERFAHREN UND VERWENDUNG DAVON
PANSEMENT DE PLAIE TRÈS HYGROSCOPIQUE ET PROCÉDÉ DE PRÉPARATION ET D'UTILISATION DE CELUI-CI

(30) Priority: 29.10.2012 CN 201210421630
(43) Date of publication of application: 29.07.2015
(73) Proprietor: Foshan United Medical Technologies Ltd, Guangdong 528225 (CN)
(72) Inventor: WANG, Xiaodong, Foshan Guangdong 528225 (CN); MO, Xiaohui, Foshan Guangdong 528225 (CN)
(74) Representative: Wilson Gunn
(86) International application number: PCT/CN2013/082801
(87) International publication number: WO 2014/067342

(56) References cited:
- WO-A1-96/14453
- WO-A1-98/46818
- WO-A2-03/033041
- CN-A- 102 580 136
- CN-A- 102 743 786
- CN-A- 103 120 804
- CN-U- 201 643 067
- CN-U- 202 875 880
- US-A- 5 674 524
- US-A1- 2009 287 130

## Description

### Filed:

This invention discloses an absorbent wound dressing and its method of manufacturing.

### Background:

It is well known that advanced wound dressings are preferred in the management of chronic wounds, particularly the gelling fibrous wound dressings such as alginate, chitosan and CMC wound dressings. These dressings have very good absorption and fluid retention properties, can provide moist environment for the wounds. However all these dressings have a common weakness, i.e. the strength of the dressing is very low, particularly the wet strength. By wet strength, we mean the dressing strength when the dressing is soaked in wound fluid or saline solutions. The low wet strength can cause many problems in the dressing changes, such as being difficult to remove as one piece which may also cause infections of the surrounding healthy skin.

Normally, all chronic wounds produce wound exudates at some stage of healing. Sometimes, the volume of the wound exudate could be as high as 50 ml per 24 hrs. Therefore there is a need in clinics for an absorbent fibrous wound dressing to remove these exudates. At the same time, it is required that the dressing should have a good wet strength so that the dressing can still be removed intact when it is saturated with wound exudates. An alginate dressing could be a good example to elaborate this. Typically an alginate dressing can absorb wound fluid up to 20 times its own weight. Commonly the largest alginate dressing is 10x20 cm with a weight of 2-3 grams. After full absorption, the dressing weight could be as high as 60 g or more. This requires the dressing's wet strength to be 60g (∼0.6N) or higher, so that when the dressing is lifted at the dressing change, the dressing will not be pulled apart by its own weight.

WO 2009/0287130 has tried in this direction. It uses some stitch bonding to re-enforce the CMC wound dressing (Aquacel). When the dressing absorbs wound fluid, the stitch bonding threads act as the re-enforcement to "hold" the dressing together, making a one piece removal possible. However as the stitch bonding threads (which is made of non-gelling fibers) are shown on the surface of the dressing, the non-gelling material may cause the threads to stick to the wound.

WO 98/46818 relates to a knitted wound dressing comprising gel forming fibers knitted onto a support yarn. The support fabric can be made of cellulosic fibers, such as viscose, cotton, polypropylene or polyester. The gelling fibers can be made of carboxymethylcellulose or alginate. An antimicrobial agent might be included into the wound dressing.

### Invention:

The present invention provides an absorbent wound dressing. The wound dressing is a knitted fabric comprising of a fluffy layer of gelling fibres and a layer of non-gelling fibres as the backing, the gelling fibres are knitted onto the backing structure and the length of the free fibre outside the backing structure is between 1-100 mm, preferably 5-50 mm. The "length of the free fibre" in this invention refers to the average length of the gelling fibres that are outside the knitting structure.

The absorbency of the dressing of the present invention shall be at least 6 g/100cm2 or higher according to the test method provided in EN 13726-1:2002 /AC:2003. When used in the management of chronic wounds, as only the fluffy gelling fibres are in contact with the wound bed which absorbs the wound fluid and the backing layer (made from non-gelling fibres) is not significantly weakened by the absorption of the fluid, the dressing is still strong and shall remain intact at dressing change.

Furthermore, when the dressing is cut in CD (cross machine) direction, the dressing of the present invention curls into a fabric roll with the backing layer inside and the fluffy layer outside. This is ideal for the management of cavity wounds, so that the fluffy layer at the outside of the fabric roll can absorb wound exudates whilst the backing layer at the inside of the fabric roll is kept away from the wound bed and can still provide strength to allow an intact removal of the dressing.

According to the present invention, the linear density of the gelling fibre is 1-10 dtex, preferably 2-5 dtex.

According to the present invention, the fibre length of the gelling fibre is 10-120mm, preferably 10-75mm.

According to the present invention, the linear density of the non-gelling yarn/filament is 50-500 dtex, preferably 50-200dtex.

The gelling fibre in the present invention refers to fibres that can form gels when absorbing water or saline or Solution A (contains 8.298 g of Sodium Chloride and 0.368g of Calcium Chloride Dihydrate as defined in British Pharmacopoeia 1995). Internationally Solution A is used in the measurement of the dressing's absorbency and wet strength, this is because the Solution A is designed to mimic the sodium and calcium content of the wound fluid. The gelling fibre has a very high absorption capacity; typically it can absorb as much as 6g of the fluid or more per gram of the gelling fibre. This kind of fibre also expands laterally enormously, sometimes; the fibre diameter can be expanded into several times or several tens of times the original value due to the absorption of water into the fibre structure (instead of being held between the fibres). Sometimes, the gelling fibre can become an amorphous gel, making it more absorbent and weak while wet. The gelling fibres in the present invention can be selected from alginate fibres, chemical modified cellulose fibres, and chemically modified chitosan fibre, such as acylated chitosan fibre, or their blends.

The said alginate fibres are calcium or calcium/sodium alginate fibres which have an absorbency of 10 g/g or above. In addition to the absorption and gelling properties, the alginate fibre can also donate calcium ions which may help wound healing and the control of bleeding.

The said chemically modified cellulose fibres are carboxymethyl cellulose or water insoluble sulfonated cellulose fibers. These fibres are originally cellulose fibres such as viscose or lyocell fibres, but have been additionally chemically modified. The modification has added a water absorbing unit to the molecular structure of the fibre which causes the fibre very absorbent and easy to gel. The degree of substitution (DS) of these fibres is typically controlled to between 0.05-0.4.

The chemically modified chitosan fibres are carboxymethyl chitosan fibres or acylated chitosan fibres. Similar to the modification to the cellulose fibres, the chemical structure of the chitosan fibre has also been modified, which has made the fibre very absorbent and easy to gel. The degree of substitution (DS) of these fibres is typically controlled to between 0.1-0.4.

The non-gelling fibrous yarn or filament can be selected from polyester, nylon, polyvinyl alcohol (PVA), viscose, Lyocell, non-gelling chitosan, Polyurethane, Polyethylene, polypropylene, silk yarn, cotton yarn and other non-gelling natural or chemical fibrous yarn of filament, or their blend.

According to the present invention, part or all gelling fibres and/or part or all non-gelling fibrous yarn or filament contain antimicrobial agent. The typical antimicrobial agent for the gelling fibres can be selected from silver, iodine, honey or PHMB. The typical antimicrobial agents for the non-gelling fibres is Silver Sodium Hydrogen Zirconium Phosphate such as Alphasan which can be added to and mixed with the polymer before extrusion therefore making antimicrobial fibres or filaments. Another method is to add silver particles such as silver compound or nano silver metal particles into the polymer solution so that silver particles exist inside the fibre/filament structures and on the surface of the fibre/filament. The wound dressing made from antimicrobial gelling fibres or antimicrobial non-gelling fibres/filaments can be used to manage the infected wounds or wounds which are at risks of infection. Typically such a wound dressing can be applied on wounds for up to 7 days without having to change the dressing. This can reduce the cost and pain for the patient.

According to the present invention, one or both ends of the gelling fibre can be fixed onto the backing layer through the loops of the backing fabric. The density of the backing fabric can be changed by the gauges (thickness) of the needles and by thickness of the filament/yarn. Typically the linear density of the filament/yarn of the backing fabric can be around 167 dtex. It can be single yarn/filament or double. The density of the backing fabric is typically around 20/inch.

According to the present invention, the fluffy layer of the dressing comprises of two or more gelling fibres.

The wound dressing of the present invention can be knitted through a circular knitting machine. The filament/yarn for the backing layer can be fed into the machine through a guiding device. The gelling fibre for the fluffy layer can be made into sliver first then into a carding/feeding device. The typical circular knitting machine is WHCW-T/S18C-27A-1176 which has two basic designs, one is to apply the blow from the front and the other is from the back. The front blow can make one end of the gelling fibre gripped by the backing layer; the back blow can make both ends of the gelling fibre into the loops of the backing layer. The free fibre length of the gelling fibre of the fabric made by the front blow method is slightly longer than that by the back blow method. The former can be 20 mm or longer, the latter is normally 3-10 m. The average free fibre length of the fabric can also be controlled by cutting which can cut the free fibre to a shorter length such as 1-5 mm.

It is well known that the wet/dry strengths of nonwoven fabrics and their wound dressings are stronger in the cross machine (CD) direction than in the machine direction (MD). Generally, during the dressing change, the dressing will break in the weakest direction.

By combining the knitting technology and the gelling and non-gelling fibres in a very structured way, the wound dressing made from the present invention has very good absorption and fluid retention properties due to the employment of gelling fibres in the fluffy layer. Also because the backing layer is made from non-gelling fibres, yarns or filaments, the dressing's wet strength is not significantly weakened by the absorption of moisture. This has given the wound dressing from the present invention the high absorbency of the gelling fibre and the high wet strength of non-gelling fibres.

The backing layer of the present invention is knitted from non-gelling yarns or filaments. The knitted structure normally has a very good elasticity and strength. The strength of the non-gelling yarns or filament is normally unaffected by the absorption of moisture therefore the strength of the backing layer of the present invention will not reduce significantly when wet, therefore making it possible for an intact removal of the dressing.

Additionally the present invention discloses a method for manufacturing a wound dressing with high absorption capacity and wet strength. The method includes:
1) Feed the non-gelling yarn or filament into the circular knitting machine to form the backing fabric.
2) Feed the gelling fibre sliver into the feed device of the circular knitting machine.
3) Apply the front blow or back blow to make one end or both ends of the gelling fibre held by the loops of the backing layer.
4) Process the fabric further through cutting and calendaring.
5) Convert the fabric into a dressing through cutting to size, packing into pouches and sterilisation (gamma irradiation or EtO or autoclave).

In step 2 of the above process, the number of slivers fed into the circular knitting machine can be varied to control the weight of the fluffy layer. For example, for a circular knitting machine that takes maximum 18 slivers, the actual number of sliver can be 6, or 9, or 12, or 18. The linear density of the sliver can be between 4-18 g/m, preferably 6-12 g/m.

In step 1 of the above process, the distance between the needles (cross machine) can be 6-18 needles/inch. The density of the backing layer is normally 12-42/inch.

The length of the free fibre of the fluffy layer depends on the method of the blow. The front blow makes the free fibre length longer, the back blow makes the free fibre length shorter. Typically the free fibre length is between 5-10 mm. If a fibre length cutting step is employed, the free fibre length can be further reduced to 1-2 mm.

The wound dressing from the present invention can be used in the management of chronic wounds, such as venous stasis ulcers, pressure ulcers, diabetic foot ulcers and other hard-to-heal wounds. Also the wound dressing of the present invention can be used in the management of cavity wounds.

### Drawings

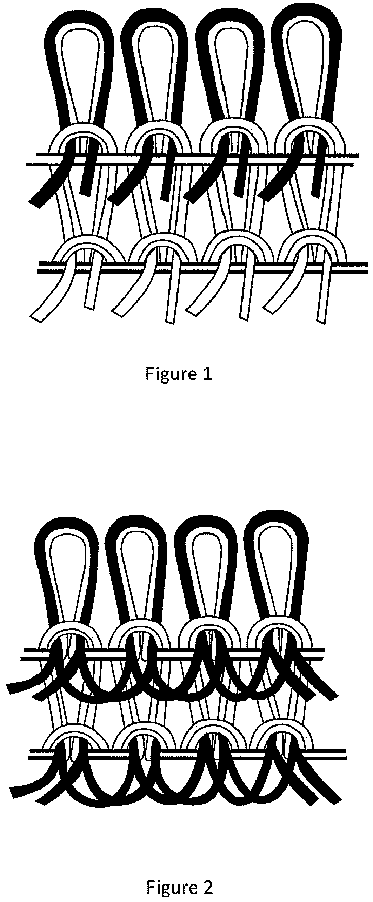

Fig 1: An illustration of the knitted fabric showing one end of the gelling fibers knitted onto the backing layer.
Fig 2: An illustration of the knitted fabric showing both ends of the gelling fibers knitted onto the backing layer.

### Examples

### Example 1:

Feed 2x183 dtex Lyocell filaments into the WHCW-S18C-24B-1056 circular knitting machine. The needle gauge is 14 needles/inch, the backing layer density is 18/inch.

Feed the calcium alginate slivers (linear density 6.7 g/m) into the above knitting machine. The fiber linear density is 2.65 dtex and the number of slivers is 18.

The blow method: front.

The fabric obtained has the weight of 410 g/m2.

The length of free fiber of the fluffy layer is 40 mm. The absorbency measured by EN 13726-1:2002 /AC:2003 is 45 g/100cm2.

The fabric dry strength in CD is 150 N/cm, Elongation 90%. The fabric wet strength 145 N/cm, elongation 93%. The fabric dry strength in CD is 41 N/cm, Elongation 266%. The fabric wet strength 40 N/cm, elongation 240%.

### Example 2:

Feed 2x167 dtex polyester filaments into the WHCW-S18C-27A-1176 circular knitting machine. The needle gauge is 14 needles/inch, the backing layer density is 20/inch.

Feed the calcium/sodium alginate slivers (linear density 6.7 g/m) into the above knitting machine. The fiber linear density is 2.65 dtex and the number of slivers is 9.

The blow method: back.

The fabric obtained has the weight of 320 g/m2.

The length of free fiber of the fluffy layer is 12 mm. The absorbency measured by EN 13726-1:2002 /AC:2003 is 32 g/100cm2.

The fabric dry strength in CD is 109 N/cm, Elongation 128%. The fabric wet strength 100 N/cm, elongation 115%. The fabric dry strength in CD is 44 N/cm, Elongation 192%. The fabric wet strength 41 N/cm, elongation 168%.

### Example 3:

Feed 2x167 dtex polyester filaments into the WHCW-S18C-27A-1176 circular knitting machine. The needle gauge is 14 needles/inch, the backing layer density is 20/inch.

Feed the carboxymethyl cellulose fiber sliver (linear density 6 g/m) into the above knitting machine. The fiber linear density is 2.1 dtex and the number of slivers is 9.

The blow method: back.

The fabric obtained has the weight of 300 g/m2.

The length of free fiber of the fluffy layer is 8 mm. The absorbency measured by EN 13726-1:2002 /AC:2003 is 33 g/100cm2.

The fabric dry strength in CD is 105 N/cm, Elongation 120%. The fabric wet strength 95 N/cm, elongation 105%. The fabric dry strength in CD is 42 N/cm, Elongation 182%. The fabric wet strength 39 N/cm, elongation 165%.

### Example 4:

Feed 2x167 dtex polyester filaments into the WHCW-S18C-27A-1176 circular knitting machine. The needle gauge is 14 needles/inch, the backing layer density is 20/inch.

Feed ancylated chitosan slivers (linear density 6 g/m) into the above knitting machine. The fiber linear density is 2.2 dtex and the number of slivers is 9.

The blow method: back.

The fabric obtained has the weight of 290 g/m2.

The length of free fiber of the fluffy layer is 5 mm. The absorbency measured by EN 13726-1:2002 /AC:2003 is 32 g/100cm2.

The fabric dry strength in CD is 101 N/cm, Elongation 128%. The fabric wet strength 92 N/cm, elongation 112%. The fabric dry strength in CD is 39 N/cm, Elongation 180%. The fabric wet strength 40 N/cm, elongation 176%.

### Example 5:

Feed 2x167 dtex polyester filaments into the WHCW-S18C-27A-1176 circular knitting machine. The needle gauge is 14 needles/inch, the backing layer density is 20/inch.

Feed calcium alginate slivers (linear density 6.7 g/m) into the above knitting machine. The fiber linear density is 2.65 dtex and the number of slivers is 9.

The blow method: back.

The fabric obtained has the weight of 300 g/m2.

The length of free fiber of the fluffy layer is 8 mm. The absorbency measured by EN 13726-1:2002 /AC:2003 is 30 g/100cm2.

The fabric dry strength in CD is 106 N/cm, Elongation 116%. The fabric wet strength 197 N/cm, elongation 103%. The fabric dry strength in CD is 41 N/cm, Elongation 180%. The fabric wet strength 37 N/cm, elongation 159%.

### Example 6:

Feed 2x167 dtex polyester filaments into the WHCW-S18C-27A-1176 circular knitting machine. The needle gauge is 14 needles/inch, the backing layer density is 20/inch.

Feed silver alginate slivers (linear density 6.7 g/m) into the above knitting machine. The fiber linear density is 2.75 dtex and the number of slivers is 9.

The blow method: back.

The fabric obtained has the weight of 320 g/m2.

The length of free fiber of the fluffy layer is 12 mm. The absorbency measured by EN 13726-1:2002 /AC:2003 is 31 g/100cm2.

The fabric dry strength in CD is 110 N/cm, Elongation 120%. The fabric wet strength 105 N/cm, elongation 113%. The fabric dry strength in CD is 39 N/cm, Elongation 190%. The fabric wet strength 42 N/cm, elongation 170%.

### Example 7:

Feed 2x167 dtex polyester filaments into the WHCW-S18C-27A-1176 circular knitting machine. The needle gauge is 14 needles/inch, the backing layer density is 20/inch.

Feed PHMB alginate slivers (linear density 6.7 g/m) into the above knitting machine. The fiber linear density is 2.75 dtex and the number of slivers is 9.

The blow method: back.

The fabric obtained has the weight of 320 g/m2.

The length of free fiber of the fluffy layer is 12 mm. The absorbency measured by EN 13726-1:2002 /AC:2003 is 31 g/100cm2.

The fabric dry strength in CD is 101 N/cm, Elongation 124%. The fabric wet strength 98 N/cm, elongation 114%. The fabric dry strength in CD is 45 N/cm, Elongation 188%. The fabric wet strength 40 N/cm, elongation 166%.

### Example 8:

Feed 2x167 dtex polyester filaments into the WHCW-S18C-27A-1176 circular knitting machine. The needle gauge is 14 needles/inch, the backing layer density is 20/inch.

Feed silver carboxymethyl cellulose slivers (linear density 6 g/m) into the above knitting machine. The fiber linear density is 2.2 dtex and the number of slivers is 9.

The blow method: back.

The fabric obtained has the weight of 300 g/m2.

The length of free fiber of the fluffy layer is 8 mm. The absorbency measured by EN 13726-1:2002 /AC:2003 is 32 g/100cm2.

The fabric dry strength in CD is 103 N/cm, Elongation 118%. The fabric wet strength 96 N/cm, elongation 104%. The fabric dry strength in CD is 38 N/cm, Elongation 176%. The fabric wet strength 35 N/cm, elongation 143%.

### Example 9:

Feed 2x167 dtex polyester filaments into the WHCW-S18C-27A-1176 circular knitting machine. The needle gauge is 14 needles/inch, the backing layer density is 20/inch.

Feed silver ancylated chitosan slivers (linear density 6 g/m) into the above knitting machine. The fiber linear density is 2.3 dtex and the number of slivers is 9.

The blow method: back.

The fabric obtained has the weight of 300 g/m2.

The length of free fiber of the fluffy layer is 5 mm. The absorbency measured by EN 13726-1:2002 /AC:2003 is 31 g/100cm2.

The fabric dry strength in CD is 98 N/cm, Elongation 125%. The fabric wet strength 90 N/cm, elongation 117%. The fabric dry strength in CD is 38 N/cm, Elongation 185%. The fabric wet strength 44 N/cm, elongation 186%.

## Claims

1. An absorbent wound dressing wherein the said wound dressing is a knitted fabric comprising of a fluffy layer of gelling fibres and a layer of non-gelling fibres as the backing and the gelling fibres are knitted onto the backing structure and the length of the free fibre outside the backing structure is between 1-100 mm, preferably 5-50 mm.

2. An absorbent wound dressing according to claim 1 **characterised in that** the said wound dressing has an absorption capacity of 6 g/100 cm² or more when tested according to EN 13726-1:2002/AC:2003.

3. An absorbent wound dressing according to claim 1 **characterised in that** the said wound dressing, when cut in the cross machine direction, curls by itself into a fabric roll with the backing layer inside and the fluffy layer outside.

4. An absorbent wound dressing according to claim 1 **characterised in that** the fibre linear density of the gelling fibre is 1-10 dtex, preferably 2-5 dtex.

5. An absorbent wound dressing according to claim 1 **characterised in that** the fibre length of the gelling fibre is 10-120 mm, preferably 10-75 mm.

6. An absorbent wound dressing according to claim 1 **characterised in that** the fibre linear density of the non-gelling fibre yarn or filament is 50-500 dtex, preferably 50-200 dtex.

7. An absorbent wound dressing according to claim 1 wherein the gelling fibres are selected from alginate fibres, chemically modified cellulose fibres, chitosan fibres or their blends.

8. An absorbent wound dressing according to claim 7 **characterised in that** the alginate fibres are calcium alginate or calcium/sodium alginate fibres.

9. An absorbent wound dressing according to claim 7 **characterised in that** the chemically modified cellulose fibres are carboxymethyl cellulose fibres or water insoluble sulfonated solvent spun cellulose fibre.

10. An absorbent wound dressing according to claim 7 **characterised in that** the chitosan fibres are carboxymethyl chitosan fibres or acylated chitosan fibres.

11. An absorbent wound dressing according to claim 1 wherein the non-gelling fibres are selected from polyester filaments or yarns, nylon filaments or yarns, PVA filaments or yarns, viscose filaments or yarns, Lyocell filaments or yarns, non-gelling chitosan filaments or yarns, polyurethane filaments or yarns, polypropylene filaments or yarns, cotton yarns, other non-gelling chemical or natural filaments or yarns, or the combination of the said filaments or yarns.

12. An absorbent wound dressing according to claim 1 wherein all or part of gelling fibres and/or all or part non-gelling fibres contain antimicrobial agents.

13. An absorbent wound dressing according to claim 1 wherein one end or both ends of the gelling fibres are knitted into the backing layer fabric.

14. An absorbent wound dressing according to claim 1 wherein the said fluffy layer comprises two or more gelling fibres.

15. A method of manufacturing the absorbent wound dressing according to claims 1-14 comprising the following steps:
1) feeding non-gelling fibre yarn or filament into the circular knitting machine to form the backing layer;
2) feeding the gelling fibre slivers into the circular knitting machine;
3) through the blow from the front or from the back knitting the gelling fibres into the backing layer;
4) processing the fabric through cutting, calendaring etc;
5) cutting and packing the fabric into pouches, and sterilising the dressing by gamma irradiation, EtO or autoclave.

## Patentansprüche

1. Absorbierender Wundverband, wobei der Wundverband ein gewirkter Stoff ist, welcher eine flaumige Schicht aus Gelierfasern und eine Schicht aus Nichtgelierfasern als Unterstützung aufweist, und die Gelierfasern auf die Stützstruktur gewirkt sind und die Länge der freien Fasern außerhalb der Stützstruktur zwischen 1 bis 100 mm, vorzugsweise 5 bis 50 mm, beträgt.

2. Absorbierender Wundverband nach Anspruch 1,
**dadurch gekennzeichnet,**
**dass** der Wundverband ein Absorptionsvermögen von 6g/100 cm² oder mehr aufweist, gemäß Test nach EN 13726-1:2002/AC:2003.

3. Absorbierender Wundverband nach Anspruch 1,
**dadurch gekennzeichnet,**
**dass** sich der Wundverband beim Schneiden in Maschinenquerrichtung selbst zu einer Stoffrolle einrollt, wobei die Stützschicht innen und die flaumige Schicht außen liegt.

4. Absorbierender Wundverband nach Anspruch 1,
**dadurch gekennzeichnet,**
**dass** die Faserfeinheit der Gelierfasern 1 bis 10 dtex, vorzugsweise 2 bis 10 dtex beträgt.

5. Absorbierender Wundverband nach Anspruch 1,
**dadurch gekennzeichnet,**
**dass** die Faserlänge der Gelierfaser 10 bis 120 mm, vorzugsweise 10 bis 75 mm beträgt.

6. Absorbierender Wundverband nach Anspruch 1,
**dadurch gekennzeichnet,**
**dass** die Faserfeinheit des Garns oder Fadens aus Nichtgelierfasern 50 bis 500 dtex, vorzugsweise 50 bis 200 dtex beträgt.

7. Absorbierender Wundverband nach Anspruch 1,
wobei die Gelierfasern ausgewählt sind aus Alginatfasern, chemisch modifizierten Zellulosefasern, Chitosanfasern oder Mischungen hiervor.

8. Absorbierender Wundverband nach Anspruch 7,
**dadurch gekennzeichnet,**
**dass** die Alginatfasern Kalziumalginat- oder Kalzium-/Sodium-Alginatfasern sind.

9. Absorbierender Wundverband nach Anspruch 7,
**dadurch gekennzeichnet,**
**dass** die chemisch modifizierten Zellulosefasern Carboxymethyl-Zellulosefasern oder wasserunlösliche Sulfat-löslich gesponnene Zellulosefasern sind.

10. Absorbierender Wundverband nach Anspruch 7,
**dadurch gekennzeichnet,**
**dass** die Chitosanfasern Carboxymethyl-Chitosanfasern oder acyliierte Chitosanfasern sind.

11. Absorbierender Wundverband nach Anspruch 1,
wobei die Nichtgelierfasern ausgewählt sind aus Polyesterfäden oder -garne, Nylonfäden oder -garne, PVA-Fäden oder -Garne, Viskosefäden oder -garne, Lyocell-Fäden oder -Garne, Nichtgelier-Chitosanfäden oder -garne, Polyurethanfäden oder -garne, Polypropylenfäden oder -garne, Baumwollgarne, andere chemische oder natürliche Nichtgelier-Fäden oder -Garne, oder der Kombination aus den genannten Fäden oder Garnen.

12. Absorbierender Wundverband nach Anspruch 1,
wobei die Gelierfasern und/oder die Nichtgelierfasern insgesamt oder teilweise antimikrobielle Wirkstoffe enthalten.

13. Absorbierender Wundverband nach Anspruch 1,
wobei ein Ende oder beide Enden der Gelierfasern in den Stützschichtstoff eingewirkt sind.

14. Absorbierender Wundverband nach Anspruch 1,
wobei die flaumige Schicht zwei oder mehr Gelierfasern aufweist.

15. Verfahren zum Herstellen eines absorbierenden Wundverbandes nach einem der Ansprüche 1 bis 14 mit den nachfolgenden Verfahrensschritten:
1) Zuführen eines Nichtgelierfasergarns oder -fadens in eine Rundstrickmaschine zum Herstellen der Stützschicht;
2) Zuführen der Gelierfaserstückchen in die Rundstrickmaschine;
3) Einwirken der Gelierfasern in die Stützschicht durch Blasen von vorne oder von hinten;
4) Verarbeiten des Stoffes durch Schneiden, Kalibrieren etc;.
5) Schneiden und Verpacken des Stoffes in Beuteln und Sterilisieren des Verbandes durch Gammabestrahlung, EtO oder Autoklavieren.

## Revendications

1. Pansement pour plaie absorbant dans lequel ledit pansement pour plaie est un tissu tricoté composé d'une couche pelucheuse de fibres gélifiantes et d'une couche de fibres non gélifiantes en tant que support et les fibres gélifiantes sont tricotées sur la structure de support et la longueur de la fibre libre à l'extérieur de la structure de support est de 1-100 mm, de préférence de 5-50 mm.

2. Pansement pour plaie absorbant selon la revendication 1 **caractérisé en ce que** ledit pansement pour plaie présente une capacité d'absorption de 6 g/100 cm² ou plus quand il est testé selon la norme EN 13726-1:2002/AC:2003.

3. Pansement pour plaie absorbant selon la revendication 1 **caractérisé en ce que** ledit pansement pour plaie, quand il est découpé dans la direction transversale de machine, gondole sur lui-même en un rouleau de tissu, avec la couche de support à l'intérieur et la couche pelucheuse à l'extérieur.

4. Pansement pour plaie absorbant selon la revendication 1 **caractérisé en ce que** la densité linéaire de fibre de la fibre gélifiante est de 1-10 dtex, de préférence de 2-5 dtex.

5. Pansement pour plaie absorbant selon la revendication 1 **caractérisé en ce que** la longueur de fibre de la fibre gélifiante est de 10-120 mm, de préférence de 10-75 mm.

6. Pansement pour plaie absorbant selon la revendication 1 **caractérisé en ce que** la densité linéaire de fibre du fil ou filament de fibre non gélifiante est de 50-500 dtex, de préférence de 50-200 dtex.

7. Pansement pour plaie absorbant selon la revendication 1 dans lequel les fibres gélifiantes sont sélectionnées parmi des fibres d'alginate, des fibres de cellulose chimiquement modifiée, des fibres de chitosane ou leurs mélanges.

8. Pansement pour plaie absorbant selon la revendication 7 **caractérisé en ce que** les fibres d'alginate sont des fibres d'alginate de calcium ou d'alginate de calcium/sodium.

9. Pansement pour plaie absorbant selon la revendication 7 **caractérisé en ce que** les fibres de cellulose chimiquement modifiée sont des fibres de cellulose de carboxyméthyle ou des fibres de cellulose filées à solvant sulfoné insoluble dans l'eau.

10. Pansement pour plaie absorbant selon la revendication 7 **caractérisé en ce que** les fibres de chitosane sont des fibres de chitosane de carboxyméthyle ou des fibres de chitosane acylaté.

11. Pansement pour plaie absorbant selon la revendication 1 dans lequel les fibres non gélifiantes sont sélectionnées parmi des filaments ou fils de polyester, des filaments ou fils de nylon, des filaments ou fils de PVA, des filaments ou fils de viscose, des filaments ou fils de Lyocell, des filaments ou fils de chitosane non gélifiant, des filaments ou fils de polyuréthane, des filaments ou fils de polypropylène, des fils de coton, d'autres filaments ou fils naturels ou chimiques non gélifiants, ou la combinaison desdits filaments ou fils.

12. Pansement pour plaie absorbant selon la revendication 1 dans lequel la totalité ou une partie de fibres gélifiantes et/ou la totalité ou une partie de fibres non gélifiantes contiennent des agents antimicrobiens.

13. Pansement pour plaie absorbant selon la revendication 1 dans lequel une extrémité ou les deux extrémités des fibres gélifiantes est/sont tricotée(s) dans le tissu de couche de support.

14. Pansement pour plaie absorbant selon la revendication 1 dans lequel ladite couche pelucheuse comprend deux, ou plus, fibres gélifiantes.

15. Procédé de fabrication du pansement pour plaie absorbant selon les revendications 1-14 comprenant les étapes suivantes :
1) l'introduction d'un fil ou filament de fibres non gélifiantes dans la machine à tricoter circulaire pour former la couche de support ;
2) l'introduction des rubans de fibres gélifiantes dans la machine à tricoter circulaire ;
3) par le soufflage depuis l'avant ou depuis l'arrière, le tricotage des fibres gélifiantes dans la couche de support ;
4) le traitement du tissu par découpe, calandrage, etc. ;
5) la découpe et le conditionnement du tissu dans des poches, et la stérilisation du pansement par rayonnement gamma, EtO ou autoclave.
